(19) Europäisches Patentamt / European Patent Office / Office européen des brevets

(11) **EP 3 261 698 B1**

(12) **EUROPÄISCHE PATENTSCHRIFT**

(45) Veröffentlichungstag und Bekanntmachung des Hinweises auf die Patenterteilung:
**26.06.2019 Patentblatt 2019/26**

(21) Anmeldenummer: **16704266.2**

(22) Anmeldetag: **15.02.2016**

(51) Int Cl.:
***A61M 16/00*** *(2006.01)*

(86) Internationale Anmeldenummer:
**PCT/EP2016/053189**

(87) Internationale Veröffentlichungsnummer:
**WO 2016/134999 (01.09.2016 Gazette 2016/35)**

(54) **BEATMUNGSVORRICHTUNG**

RESPIRATORY DEVICE

DISPOSITIF RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.02.2015 DE 102015203455**

(43) Veröffentlichungstag der Anmeldung:
**03.01.2018 Patentblatt 2018/01**

(73) Patentinhaber: **Hamilton Medical AG
7402 Bonaduz (CH)**

(72) Erfinder:
• **KÜHN, Lars
7214 Grüsch (CH)**
• **NOVOTNI, Dominik
7000 Chur (CH)**
• **LAUBSCHER, Thomas
7403 Rhäzüns (CH)**

(74) Vertreter: **Ruttensperger Lachnit Trossin Gomoll Patent- und Rechtsanwälte PartG mbB Postfach 20 16 55 80016 München (DE)**

(56) Entgegenhaltungen:
**US-A1- 2009 007 915     US-A1- 2012 272 961
US-A1- 2012 298 108     US-A1- 2013 074 844**

**Beschreibung**

Die vorliegende Erfindung betrifft eine Beatmungsvorrichtung zur wenigstens unterstützend-teilweisen künstlichen Beatmung von Patienten, insbesondere von humanen Patienten. Die Beatmungsvorrichtung weist eine Atemgas-Leitungsanordnung, eine Druckveränderungsanordnung und eine Steuereinrichtung auf. Die Druckveränderungsanordnung dient der Veränderung des Drucks von Atemgas in der Atemgas-Leitungsanordnung während des Beatmungsbetriebs der Beatmungsvorrichtung. Die Steuereinrichtung dient der Steuerung des Betriebs der Beatmungsvorrichtung, insbesondere der Druckveränderungsanordnung. Die Steuereinrichtung hat einen Dateneingang zur Übertragung von Betriebs- oder/und Patientendaten an die Steuereinrichtung. Sie ist dazu ausgebildet, einen Beatmungs-Betriebsparameter für den Betrieb der Beatmungsvorrichtung, insbesondere der Druckveränderungsanordnung, selektiv mittels eines vorbestimmten ersten Datenzusammenhangs oder mittels eines vom ersten verschiedenen vorbestimmten zweiten Datenzusammenhangs zu ermitteln.

Derartige Beatmungsvorrichtungen sind aus der klinischen Anwendung, etwa in der Chirurgie oder in der Intensivmedizin, zur künstlichen Beatmung von Personen bekannt. Die Beatmungsvorrichtungen können dazu eingesetzt werden, Personen vollständig zu beatmen, die dazu aus eigener Kraft nicht mehr in der Lage sind. Hierzu zählen Patienten mit narkotisiertem Atemapparat oder komatöse Patienten. Die vorliegende Anmeldung betrifft daher neben Intensiv-Beatmungsvorrichtungen auch, aber nicht nur, Anästhesie-Beatmungsvorrichtungen. Beatmungsvorrichtungen der eingangs genannten Art können jedoch auch lediglich zur unterstützenden Beatmung von Personen verwendet werden, die zwar aus eigener Kraft in einem gewissen Maß zur Atmung in der Lage sind, jedoch dabei ihren Atemgasbedarf selbstständig nicht vollständig decken können.

Neben der Humanmedizin sind Beatmungsvorrichtungen der vorliegenden Erfindung auch in der Veterinärmedizin im Einsatz, etwa zur Beatmung von Tieren während Narkosephasen. Auch derartige veterinärmedizinisch behandelte Tiere sollen "Patienten" im Sinne der vorliegenden Anmeldung sein.

Mit der Atemgas-Leitungsanordnung wird das Atemgas zum Patienten, insbesondere in dessen Atemorgane, geleitet. Die Atemgas-Leitungsanordnung kann wenigstens abschnittsweise auch zum Abführen von verbrauchtem, verstoffwechseltem Atemgas vom Patienten in die Atmosphäre verwendet werden. Die Atemgas-Leitungsanordnung umfasst wenigstens eine Atemgasleitung, etwa Schlauchleitung, und wenigstens ein Ventil zur selektiven Wahl von Strömungswegen für die Inspiration und die Exspiration.

Die zur künstlichen Beatmung von Patienten notwendige Atemgasströmung in der Atemgas-Leitungsanordnung wird durch die Druckveränderungsanordnung bewirkt, die dazu ausgebildet ist, den Druck des Atemgases in der Atemgas-Leitungsanordnung zu verändern, somit Druckunterschiede zwischen einer Atemgasmenge außerhalb des Körpers des Patienten und einem Gasdruck im Inneren der Atemorgane des Patienten zu erzeugen und dadurch eine Atemgasströmung zu den Atemorganen des Patienten hin oder von dem Patienten weg zu bewirken. Üblicherweise umfasst die Druckveränderungsanordnung eine Pumpe, ein Gebläse oder einen Verdichter und dergleichen zur Manipulation des Drucks des Atemgases in der Atemgas-Leitungsanordnung.

Die Druckveränderungsanordnung kann auch ein Reduzierventil umfassen. Beispielsweise kann das an ein installiertes Atemgas-Druckreservoir, wie es in Kliniken als zentrale Atemgas-Versorgungsinstallation häufig anzutreffen ist, angeschlossene Reduzierventil als Druckveränderungsanordnun im Sinne der vorliegenden Erfindung dienen. Durch das Reduzierventil kann der Druck des von der Atemgas-Versorgungsinstallation an Zapfstellen in Behandlungs- oder/und Patientenräumen abzapfbaren Atemgases auf einen für künstliche Beatmung geeigneten Druckbereich, beispielsweise mit einem Überdruckmaximum von etwa 30 mbar, gemindert werden.

Der Betrieb der Beatmungsvorrichtung, insbesondere der Druckveränderungsanordnung aber auch von Ventilen der Atemgas-Leitungsanordnung, während des Beatmungsbetriebs wird von der Steuereinrichtung gesteuert. Durch die Steuereinrichtung kann also wenigstens ein Beatmungs-Betriebsparameter für den Betrieb der Druckveränderungsanordnung selektiv mittels eines ersten Datenzusammenhangs oder eines zweiten Datenzusammenhangs, die jeweils vorbestimmt sind, ermittelt werden.

Die folgenden Dokumente sind relevanter Stand der Technik: US 2013/074844 A1, US 2012/298108 A1, US 2009/007915 A1, US 2012/272961 A1. Eine gattungsgemäße Beatmungsvorrichtung ist aus der WO 2007/085108 A bekannt. Diese Druckschrift, die ganz allgemein die Bereitstellung einer möglichst optimalen Beatmung eines Patienten zur Aufgabe hat, lehrt unter anderem, abhängig von einer nicht näher bestimmten Aktivität des künstlich zu beatmenden Patienten ein Tidalvolumen und eine geeignete Beatmungsfrequenz als Beatmungs-Betriebsparameter entweder nach der bekannten Formel von Otis oder nach der bekannten Formel von Mead als den vorbekannten Datenzusammenhängen zu berechnen. Die genannte Druckschrift schweigt sich jedoch aus, gemäß welchen Kriterien abhängig von welcher Aktivität die eine oder die andere Formel verwendet werden soll.

Grundsätzlich stellen die Berechnungsformeln von Otis und Mead die seit Jahrzehnten zur Berechnung von Beatmungs-Betriebsparametern verwendeten Grundlagen dar. Da Beatmungsvorrichtungen in der medizinischen Therapie nicht nur lebenserhaltende Funktionen erfüllen, sondern bei fehlerhaft gewählten Beatmungs-Betriebsparametern über eine nicht ausreichende Funktionserfüllung hinaus eine den jeweils behandelten Patienten massiv schädigende Wirkung haben können, ist die Fachwelt - insbesondere das mit Beatmungsvorrichtungen am Patienten arbeitende ärztliche Fachpersonal - äußerst zurückhaltend in der Anwendung anderer Berechnungsformeln als der bewährten oben genannten Formeln von Otis oder Mead. Von diesen ist hinlänglich bekannt, dass sie zum funktionserfüllenden Betrieb von Beatmungsvorrichtungen geeignet sind.

Aus der WO 2013/045563 A1 ist ein Beatmungsschlauchsystem bekannt, welches auch an der vorliegenden Erfindung als Atemgas-Leitungsanordnung zum Einsatz kommen kann.

Die Aufgabe der vorliegenden Erfindung ist es, die eingangs genannte Beatmungsvorrichtung weiter zu verbessern, insbesondere dahingehend, dass diese noch präziser auf den tatsächlichen Atemgasbedarf eines Patienten abgestimmt werden kann. Diese Aufgabe wird erfindungsgemäß gelöst durch eine gattungsgemäße Beatmungsvorrichtung, bei welcher die Steuereinrichtung dazu ausgebildet ist, den Beatmungs-Betriebsparameter für einen vorgegebenen Atemgasbedarf abhängig von einem einen Beatmungswiderstand im Zusammenhang mit einem zu beatmenden Patienten anzeigenden Widerstandsdatenwert gemäß dem ersten Datenzusammenhang oder gemäß dem zweiten Datenzusammenhang zu ermitteln. Die Erfindung ist definiert durch die beigefügten Ansprüche.

Dabei ist bevorzugt daran gedacht, dass die Steuereinrichtung dazu ausgebildet ist, abhängig von einem einen Beatmungswiderstand im Zusammenhang mit einem zu beatmenden Patienten anzeigenden Widerstandsdatenwert einen Datenzusammenhang aus dem vorbestimmten ersten und dem vorbestimmten zweiten Datenzusammenhang auszuwählen und so den Beatmungs-Betriebsparameter für einen vorgegebenen Atemgasbedarf gemäß dem ausgewählten Datenzusammenhang aus erstem Datenzusammenhang und zweitem Datenzusammenhang zu ermitteln.

Ein Beatmungs-Betriebsparameter im Sinne der vorliegenden Anmeldung ist ein Parameter, welcher für den Betrieb der Beatmungsvorrichtung, insbesondere der Druckveränderungsanordnung, an dieser eingestellt wird. Patientendaten sind Daten, welche den zu beatmenden Patienten beschreiben, wie etwa Körpergewicht, Körpergröße, Krankheitsbild, Alter, Geschlecht, Körpermasseindex, Fitnessgrad und dergleichen.

Grundsätzlich erfolgt eine künstliche Beatmung stets gegen den systemimmanenten Beatmungswiderstand der Beatmungsvorrichtung und des Patienten. Die Beatmungsvorrichtung hat gegen diesen Beatmungswiderstand Arbeit zu verrichten, um die Alveolen des Patienten ausreichend mit frischem Atemgas zu versorgen. Zwar ist auch eine Arbeit gegen einen Beatmungswiderstand zur Abführung von verbrauchtem Atemgas aus den Alveolen denkbar, bevorzugt wird jedoch nur das Zuführen von frischem Atemgas zu den Alveolen des Patienten (Inspiration) von der Beatmungsvorrichtung unterstützt. Das Ausatmen (Exspiration) wird üblicherweise allein aufgrund der erhöhten Körperspannung des Atemapparats des Patienten bewirkt, die während der unmittelbar vorhergehenden Inspirationsphase durch das in den Körper eingeleitete Gas aufgebaut wurde. Hierzu wird nach dem Schließen eines Inspirationsventils, mit welchem ein frisches Atemgas zum Patienten hin führender Inspirationsschlauch abgesperrt wird, in der Regel ein Exspirationsventil geöffnet, sodass sich die Körperspannung des Atemapparats des Patienten unter Ausschieben von verbrauchtem Atemgas in die Atmosphäre entspannen kann.

Der im jeweiligen Beatmungsfall zu überwindende Beatmungswiderstand ist fallabhängig unterschiedlich. Da die Beatmungsvorrichtung üblicherweise standardisierte Bauteile verwendet, insbesondere für die Atemgas-Leitungsanordnung, werden Unterschiede im Beatmungswiderstand von zwei unterschiedlichen, mit derselben Beatmungsvorrichtung beatmeten Patienten im Wesentlichen von den Patienten selbst abhängen. Abhängig von dem jeweiligen Zustand des Patienten kann der Beatmungswiderstand größtenteils durch den Patienten verursacht sein. Dennoch sei an dieser Stelle darauf hingewiesen, dass auch die Atemgas führenden Bauteile der Beatmungsvorrichtung einen Beitrag zum Beatmungswiderstand leisten. Möglicherweise kann der Beitrag der Beatmungsvorrichtung zum Beatmungswiderstand im Einzelfall oder in einer Klasse von Fällen gegenüber dem Beitrag des Patienten zum Beatmungswiderstand unberücksichtigt bleiben, ohne dass durch diese Vereinfachung eine zu große Abweichung von der Realität eingeführt wird.

Durch die Formulierung "Beatmungswiderstand im Zusammenhang mit einem zu beatmenden Patienten" soll jedenfalls zum Ausdruck gebracht sein, dass grundsätzlich der insgesamt bei der künstlichen Beatmung zu überwindende Beatmungswiderstand maßgeblich sein soll, wenngleich dieser - wie oben ausgesagt - unter Umständen zulässigerweise vereinfacht berücksichtigt werden kann. Der verwendete Ausdruck ist gleichbedeutend mit dem Beatmungswiderstand im jeweiligen Beatmungsfall.

Der Atemgasbedarf eines Patienten ergibt sich aus seinem Stoffwechsel, für den das Atemgas zur Oxidation benötigt wird. Der Atemgasbedarf kann angegeben werden durch das Tidalvolumen, also das Atemgasvolumen eines Atemzugs, multipliziert mit der Beatmungsfrequenz, also der Anzahl an Wiederholungen von Atemzügen während einer vorbestimmten Zeitdauer, üblicherweise 1 Minute.

Ein und derselbe Atemgasbedarf kann dabei über unterschiedliche Wertepaare von Tidalvolumen und Beatmungsfrequenz realisiert werden. Eine Vergrößerung des einen Werts erfordert eine reziproke Verkleinerung des jeweils anderen Werts.

Für unterschiedliche Patientenzustände, wie etwa Krankheitsbilder, Sedierungsgrad, Erschöpfung und dergleichen, und Patientenkonstitutionen, wie etwa Geschlecht, Körperbau, Muskelmasse, Fitnessgrad und dergleichen, existieren unterschiedliche klinisch akzeptierte Richtwerte hinsichtlich des jeweils geeigneten Tidalvolumens. Diese können jedoch nur eine grobe Orientierung geben, da es für jeden Behandlungsfall stark auf die individuellen körperlichen und gesundheitlichen Verhältnisse des jeweiligen Patienten ankommt.

Der Beatmungswiderstand kann als einen Bestandteil einen Strömungswiderstand, also etwa bewirkt durch Reibung des Atemgases an den das Atemgas führenden Leitungswänden und durch Turbulenzen in der Atemgasströmung, und als einen alternativen oder zusätzlichen Bestandteil einen Körperwiderstand aufweisen, etwa bewirkt durch die Verformung der Atemgas führenden Leitungen und des Körpers des Patienten während des Einleitens von Atemgas in den Körper des Patienten. Je nach Zustand und Konstitution des jeweiligen Patienten kann sich eine unterschiedliche Gewichtung der jeweiligen Bestandteile des Beatmungswiderstands ergeben.

Es hat sich herausgestellt, dass für ein und denselben Patienten in ein und demselben Zustand die grundsätzlich akzeptierten und bewährten Formen von Otis und Mead - und eigentlich sind dies die einzigen in der Fachwelt anerkannten Formeln - zu unterschiedlichen Werten, mitunter erheblich unterschiedlichen Werten für ein und denselben Beatmungs-Betriebsparameter führen können. Dabei kommt es nach neuesten Untersuchungen der Anmelderin überraschend nicht so sehr auf die Aktivität des jeweiligen Patienten an als vielmehr auf den im Zusammenhang mit dem Patienten während einer künstlichen Beatmung verursachten Beatmungswiderstand.

Daher ist der Beatmungswiderstand im Zusammenhang mit dem jeweiligen Patienten der wohl geeignetste Parameter, einen von zwei vorbestimmten Datenzusammenhängen zur Ermittlung des für den jeweiligen Patienten und seinen konkreten Atemgasbedarf geeignetsten Beatmungs-Betriebsparameters auszuwählen.

Zwar liegen die nach Otis und Mead jeweils ermittelbaren Beatmungs-Betriebsparameter stets im zulässigen Bereich. Dennoch kann im Einzelfall der nach der einen Formel berechnete Beatmungs-Betriebsparameter vorteilhafter sein als der nach der jeweils anderen Formel berechnete Beatmungs-Betriebsparameter.

Auch wenn hier aufgrund der überragenden Bedeutung der Formeln von Otis und Mead immer wieder von diesen Formeln die Rede ist, ist die erfindungsgemäße Beatmungsvorrichtung nicht auf diese Formeln als den vorbestimmten Datenzusammenhängen beschränkt. Grundsätzlich liegt die Idee der vorliegenden Erfindung darin, aus einem vorbestimmten ersten Datenzusammenhang und einem vorbestimmten zweiten Datenzusammenhang jeweils jenen auszuwählen, welcher für die konkret vorliegende Therapiesituation der geeignetste ist bzw. zu dem geeignetsten Beatmungs-Betriebsparameter führt.

Dies schließt auch und gerade die durch die vorliegende Erfindung eröffnete Möglichkeit ein, dass ein und derselbe Patient mit ein und demselben Atemgasbedarf durch die erfindungsgemäße Beatmungsvorrichtung zu unterschiedlichen Zeiten mit nach unterschiedlichen Datenzusammenhängen ermittelten Beatmungs-Betriebsparametern beatmet wird, wenn sich während der Beatmung der zu überwindende Beatmungswiderstand ausreichend ändert. Die Änderung des Beatmungswiderstands kann beispielsweise durch teilweise Genesung oder durch medikamentöse Eingriffe bewirkt werden. Bereits eine bloße Umlagerung des Patienten kann eine Änderung des Beatmungswiderstands bewirken. Dabei kann der Patient über die Dauer der künstlichen Beatmung hinweg stets die gleiche Aktivität aufweisen, etwa wenn der Patient fortwährend im Koma liegt. Gemäß der vorliegenden Erfindung ist die Steuereinrichtung also insbesondere dazu ausgebildet, den Beatmungs-Betriebsparameter für einen vorgegebenen Atemgasbedarf ausschließlich abhängig von dem Widerstandsdatenwert, der einen Beatmungswiderstand im Zusammenhang mit dem zu beatmenden Patienten anzeigt, und unabhängig von dessen eigener Aktivität, insbesondere Atmungsaktivität, gemäß dem ersten Datenzusammenhang oder gemäß dem zweiten Datenzusammenhang zu ermitteln.

Der jeweilige Beatmungswiderstand im Zusammenhang mit dem zu beatmenden Patienten wird vorteilhaft durch einen diesen anzeigenden Widerstandsdatenwert repräsentiert, welcher in Tabellen und Grafiken Werten anderer Größen oder auch den Datenzusammenhängen zugeordnet werden kann.

Grundsätzlich kann daran gedacht sein, Daten in die Steuereinrichtung über den genannten Dateneingang einzugeben. So kann auch der Widerstandsdatenwert manuell von einem behandelnden Arzt über den Dateneingang in die Steuereinrichtung eingegeben werden. Der Dateneingang kann hierzu mit einem geeigneten Eingabegerät verbunden oder verbindbar sein, etwa einer Tastatur.

Vorteilhafterweise ist die Beatmungsvorrichtung jedoch dazu ausgebildet, den Widerstandsdatenwert im Zusammenhang mit dem jeweils zu beatmenden Patienten zu bestimmen. Hierzu kann die Beatmungsvorrichtung beispielsweise Betriebsparameter, etwa der Druckveränderungsanordnung, in ihrer zeitlichen Veränderung erfassen. Zusätzlich oder alternativ kann die Beatmungsvorrichtung mit einem oder mehreren Sensoren datenübertragungsmäßig verbunden sein, die zur Erfassung von Betriebsgrößen der Druckveränderungsanordnung oder/und der Atemgas-Leitungsanordnung ausgebildet sind. Die von diesen Sensoren ermittelten Erfassungsdaten können dann über den Dateneingang der Steuereinrichtung zugeleitet werden, welche gemäß an sich bekannter Berechnungsmodelle aus den von den Sensoren erfassten Datenwerten den aktuell vorliegenden Widerstandsdatenwert im Zusammenhang mit dem zu beatmenden Patienten bestimmen kann.

Da sich, wie oben bereits angedeutet wurde, der Beatmungswiderstand und somit der ihn anzeigende Widerstandsdatenwert mit der Zeit verändern können oder sogar mit überwiegender Wahrscheinlichkeit verändern werden, ist die Beatmungsvorrichtung gemäß einer vorteilhaften Weiterbildung der vorliegenden Erfindung dazu ausgebildet, den Widerstandsdatenwert wiederholt zu bestimmen, um die Beatmungsvorrichtung, genauer deren Druckveränderungsanordnung, mit möglichst aktuellen Daten und daraus hergeleiteten Beatmungs-Betriebsparametern zu betreiben.

Besonders bevorzugt ist die Beatmungsvorrichtung dazu ausgebildet, den Widerstandsdatenwert nach jedem oder für jeden Beatmungszyklus zu bestimmen, sodass für jeden folgenden Beatmungszyklus der erforderliche Beatmungs-Betriebsparameter mit der größtmöglichen Aktualität vorliegt.

Die Ermittlung des Beatmungs-Betriebsparameters mittels des ersten oder des zweiten Datenzusammenhangs kann ein einfacher Ermittlungsschritt unter Anwendung einer Formel auf einen Satz vorbestimmter Eingangsdaten sein. Die Ermittlung des Beatmungs-Betriebsparameters kann jedoch auch über mehrere Berechnungsstufen hinweg erfolgen. Beispielsweise kann die Beatmungsvorrichtung dazu ausgebildet sein, zunächst für einen Widerstandsdatenwert einen ersten Beatmungs-Basisbetriebsparameter nach einem vorbestimmten ersten Datenbasiszusammenhang zu bestimmen und einen zweiten Beatmungs-Basisbetriebsparameter nach einem vom ersten verschiedenen vorbestimmten zweiten Datenbasiszusammenhang zu bestimmen. Die Beatmungsvorrichtung kann weiter dazu ausgebildet sein, dann den ersten und den zweiten Beatmungs-Basisbetriebsparameter miteinander zu vergleichen und abhängig von einem Ergebnis des Vergleichs den Beatmungs-Betriebsparameter mittels des vorbestimmten ersten Datenzusammenhangs oder mittels des vorbestimmten zweiten Datenzusammenhangs zu ermitteln.

Um die bereits ermittelten Beatmungs-Basisbetriebsparameter und den zu dessen Ermittlung betriebenen Aufwand für die weitere Bestimmung des Beatmungs-Betriebsparameters nutzen zu können, ist es vorteilhaft, wenn der erste Datenzusammenhang eine Funktion ist. Dieser Funktion des ersten oder/und des zweiten Datenbasiszusammenhangs steht eine zum selben Beatmungs-Betriebsparameter führende Funktion des ersten Beatmungs-Basisbetriebsparameters oder/und des zweiten Beatmungs-Basisbetriebsparameters gleich. Beispielsweise kann der erste Datenzusammenhang eine einen Mittelwert aus dem ersten und dem zweiten Datenbasiszusammenhang bildende Funktion sein. Dem steht wiederum eine einen Mittelwert aus dem ersten und dem zweiten Beatmungs-Basisbetriebsparameter bildende Funktion gleich. Grundsätzlich kann der genannte Mittelwert jede Art von Mittelwert sein, also ein arithmetischer, geometrischer oder harmonischer Mittelwert. Für die Anwendung im Bereich der künstlichen Beatmung hat sich jedoch der arithmetische Mittelwert als besonders vorteilhaft erwiesen.

Entsprechendes gilt mutatis mutandis für den zweiten Datenzusammenhang, der ebenfalls eine Funktion des ersten oder/und des zweiten Datenbasiszusammenhangs sein kann, und insbesondere eine einen Mittelwert aus dem ersten und dem zweiten Datenbasiszusammenhang bildende Funktion sein kann. Das oben zum Mittelwert im Zusammenhang mit dem ersten Datenzusammenhang Gesagte trifft ebenso auf den zweiten Datenzusammenhang zu. Auch hier steht eine zum selben Beatmungs-Betriebsparameter führende Funktion dsm ersten Beatmungs-Basisbetriebsparameters und des zweiten Beatmungs-Basisbetriebsparameters einer Funktion des ersten oder/und des zweiten Datenbasiszusammenhangs gleich. In einer einfachen aber vorteilhaften Lösung kann der zweite Datenzusammenhang der zweite Datenbasiszusammenhang sein, sodass der zweite Beatmungs-Basisbetriebsparameter der Beatmungs-Betriebsparameter ist, wenn dieser über den zweiten Datenzusammenhang ermittelt wird.

Dann, wenn beide Datenzusammenhänge jeweils Funktionen des ersten oder/und des zweiten Datenbasiszusammenhangs sind, sind diese unterschiedliche Funktionen des ersten oder/und des zweiten Datenbasiszusammenhangs, um einen für den jeweils zu beatmenden Patienten möglichst geeigneten und bestmöglich angepassten Beatmungs-Betriebsparameter in Abhängigkeit von dem zu überwindenden Beatmungswiderstand ermitteln zu können.

Der Vergleich, den die Beatmungsvorrichtung am ersten und am zweiten Beatmungs-Basisbetriebsparameter ausführt, kann grundsätzlich ein beliebiger Vergleich sein, auch ein mehrstufiger Vergleich, in dem mehrere aus den jeweiligen Beatmungs-Basisbetriebsparametern abgeleitete Werte miteinander verglichen werden können.

Besonders bevorzugt ist eine wenig Rechenleistung erfordernde und immer wieder schnell bereitstellbare einfache Lösung, bei welcher die Beatmungsvorrichtung dazu ausgebildet ist, dann, wenn der erste Beatmungs-Basisbetriebsparameter größer als der zweite Beatmungs-Basisbetriebsparameter ist, einen Mittelwert des ersten und des zweiten Beatmungs-Basisbetriebsparameters als den Beatmungs-Betriebsparameter zu verwenden, und dann, wenn der erste Beatmungs-Basisbetriebsparameter kleiner als der zweite Beatmungs-Basisbetriebsparameter ist oder gleich dem Beatmungs-Basisbetriebsparameter ist, den zweiten Beatmungs-Basisbetriebsparameter als den Beatmungs-Betriebsparameter zu verwenden.

Wiederum ist der Mittelwert bevorzugt ein arithmetischer Mittelwert, wenngleich die oben genannten anderen Mittelwerte nicht ausgeschlossen sein sollen.

Zusätzlich oder alternativ kann ein Datenzusammenhang aus vorbestimmtem erstem und zweitem Datenzusammenhang zur Ermittlung des Beatmungs-Betriebsparameters abhängig von einem Schwellen-Widerstandsdatenwert ausgewählt werden, wenn für wenigstens einen Teil von an der Beatmungsvorrichtung auswählbaren oder/und einstellbaren Atemgasbedarfswerten jeweils ein Schwellen-Widerstandsdatenwert existiert. Dann, wenn derartige Schwellen-Widerstandsdatenwerte existieren, kann zur Auswahl eines Datenzusammenhangs vorgesehen sein, dass die Steuereinrichtung dazu ausgebildet ist, dann, wenn der Widerstandsdatenwert des zu beatmenden Patienten unterhalb des Schwellen-Widerstandsdatenwerts gelegen ist, den Beatmungs-Betriebsparameter gemäß dem ersten Datenzusammenhang zu ermitteln, und dann, wenn der Widerstandsdatenwert des konkreten Patienten oberhalb des Schwellen-Widerstandsdatenwerts gelegen ist, den Beatmungs-Betriebsparameter gemäß dem zweiten Datenzusammenhang zu ermitteln.

Wiederum kann bevorzugt der erste Datenzusammenhang eine Funktion eines ersten oder/und eines zweiten Datenbasiszusammenhangs und kann der zweite Datenzusammenhang eine vom ersten Datenzusammenhang verschiedene Funktion eines ersten oder/und eines zweiten Datenbasiszusammenhangs sein.

Die Schwellen-Widerstandsdatenwerte können auf Grundlage von durchgeführten Experimenten bestimmt werden. Beispielsweise kann ein Widerstandsdatenwert als Schwellen-Widerstandsdatenwert ermittelt werden, wenn für kleinere Widerstandsdatenwerte der eine Datenzusammenhang aus dem ersten und dem zweiten Datenzusammenhang einen für den zu beatmenden Patienten besser angepassten Beatmungs-Betriebsparameter liefert, und wenn für größere Widerstandsdatenwerte der jeweils andere Datenzusammenhang den besser angepassten Beatmungs-Betriebsparameter liefert. Als Entscheidungshilfe, welcher Beatmungs-Betriebsparameter der geeignetere ist, können die oben beschriebenen klinisch akzeptierten Richtwerte dienen. Derjenige Datenzusammenhang, welcher für den gegebenen Atemgasbedarf eines gegebenen Patienten einen Beatmungs-Betriebsparameter liefert, welcher unter Würdigung der Gesamtumstände näher an dem für den jeweiligen Patienten einschlägigen klinisch akzeptierten Richtwert liegt, ist im Zweifel der geeignetere.

Ein Schwellen-Widerstandsdatenwert für einen gegebenen Atemgasbedarf kann sich auch an einem Schnittpunkt der Graphen des ersten und des zweiten Datenzusammenhangs als jeweilige Funktionen des Beatmungs-Betriebsparameters in Abhängigkeit von den möglichen Widerstandsdatenwerten ergeben. An diesem Schnittpunkt liefern der erste und der zweite Datenzusammenhang für den gegebenen Atemgasbedarf des konkreten Patienten, also bei ansonsten gleichen Betriebs- und Patientendaten, denselben Wert für den Beatmungs-Betriebsparameter. An diesem Schnittpunkt ist es folglich gleichgültig, ob der erste oder der zweite Datenzusammenhang zur Ermittlung des Beatmungs-Betriebsparameters verwendet wird, da das Ergebnis das gleiche ist. In einem auf einer Seite des Schnittpunkts und somit des Schwellen-Widerstandsdatenwerts gelegenen Wertebereich, etwa zu kleineren Widerstandsdatenwerten hin, kann jedoch der eine Datenzusammenhang vorteilhaftere Beatmungs-Betriebsparameter liefern, in einem auf der anderen Seite des Schnittpunkts gelegenen Wertebereich der jeweils andere Datenzusammenhang.

Wie bei Beatmungsvorrichtungen allgemein üblich, ist bevorzugt der Beatmungs-Betriebsparameter ein Beatmungs-Tidalvolumen oder eine Beatmungsfrequenz. Bei bekanntem Atemgasbedarf, beispielsweise angegeben als Minutenvolumen, also als der dem Patienten während einer Minute zugeführten Atemgasmenge, - und die vorliegende Erfindung setzt den Atemgasbedarf als vorgegeben voraus - lässt sich das Beatmungs-Tidalvolumen bei bekannter Beatmungsfrequenz und lässt sich die Beatmungsfrequenz bei bekanntem Beatmungs-Tidalvolumen errechnen, da das Minutenvolumen das Produkt aus Beatmungs-Tidalvolumen und Beatmungsfrequenz ist. Bevorzugt ist der Atemgasbedarf daher ein Minutenvolumen, besonders bevorzugt angegeben in Prozent-Minutenvolumen, da aus einer bekannten prozentualen Angabe des Minutenvolumens sehr einfache Umrechnungen zu anderen Minutenvolumina möglich sind, etwa wenn sich während der Therapie der Zustand des Patienten und mit diesem dessen Atemgasbedarf ändert. Andere Beatmungs-Betriebsparameter können beispielsweise der Überdruck-Spitzenwert, der während einer Inspirationsphase im Atemgas erreicht wird, der positive end-exspiratorische Druck (PEEP), die relative Feuchte des Atemgases, die Temperatur des Atemgases und dergleichen sein.

Eingangs wurde bereits erläutert, welche konkreten Widerstandsformen zum Beatmungswiderstand im Zusammenhang mit einem Patienten beitragen können. Dieser physikalischen Tatsache folgend kann der Widerstandsdatenwert einen Strömungswiderstandswert (sogenannte "Resistance") der Atemwege des zu beatmenden Patienten oder/und der Atemgas führenden Leitungen der Beatmungsvorrichtung berücksichtigen. Etwaig in einer Leitung vorgesehene Ventile gelten als Teil der Leitung. In dem grundsätzlich möglichen, wenngleich unwahrscheinlichen Fall, dass der Strömungswiderstand den weitaus überwiegenden Teil des Gesamtwiderstands der Beatmung ausmacht, kann es unter Umständen ausreichen, nur den Strömungswiderstandswert (Resistance) des Beatmungsfalls bzw. des Patienten als Widerstandsdatenwert zu berücksichtigen.

Zusätzlich oder alternativ kann der Widerstandsdatenwert einen Nachgiebigkeitswert (sogenannte "Compliance") der Atmungsorgane des zu beatmenden Patienten oder/- und der Atemgas führenden Leitungen der Beatmungsvorrichtung berücksichtigen. Wiederum gilt für den grundsätzlich möglichen, aber unwahrscheinlichen Fall, dass der Nachgiebigkeitswert den weitaus überwiegenden Teil des Gesamtwiderstands der Beatmung ausmacht, dass die Berücksichtigung allein des Nachgiebigkeitswerts als der Widerstandsdatenwert ausreichen kann.

Ein möglichst realistisches Bild von dem Beatmungswiderstand des konkreten Beatmungsfalls, also des Beatmungswiderstands im Zusammenhang mit einem Patienten, ergibt sich jedoch dann, wenn der diesen anzeigende Widerstandsdatenwert die Resistance und die Compliance des Beatmungsfalls berücksichtigt. Daher berücksichtigt der Widerstandsdatenwert bevorzugt eine aus dem Produkt von Resistance und Compliance des Beatmungsfalls gebildete Beatmungs-Zeitkonstante. "Berücksichtigen" heißt dabei, dass der genannte Wert bzw. die genannten Werte Eingang in die Bestimmung des Widerstandsdatenwerts finden, wobei nicht ausgeschlossen sein soll, dass weitere Werte ebenfalls eine Rolle bei der Bestimmung des Widerstandsdatenwerts spielen können. Höchstbevorzugt ist der Widerstandsdatenwert jedoch die für diesen Zweck bereits im Stand der Technik häufig herangezogene Beatmungs-Zeitkonstante als Produkt aus Resistance und Compliance.

Zur Ermittlung eines Beatmungs-Betriebsparameters, insbesondere der vorgenannten konkreten Beispiele für einen Beatmungs-Betriebsparameter, existieren in der Fachwelt unterschiedliche Ansätze, die auf unterschiedlichen Hypothesen beruhen. Beispielsweise kann ein Datenbasiszusammenhang aus erstem und zweitem Datenbasiszusammenhang oder kann ein Datenzusammenhang aus erstem und zweitem Datenzusammenhang auf einer Hypothese minimalen Kraftaufwands für die Beatmung beruhen und kann der jeweils andere Datenbasiszusammenhang oder der jeweils andere Datenzusammenhang auf einer Hypothese minimaler bei der Beatmung geleisteter Arbeit beruhen. Da es grundsätzlich möglich sein kann, dass die minimal aufgewendete Kraft auch die minimale Arbeit leistet, soll für die Hypothese minimalen Kraftaufwands ausgeschlossen sein, dass an einem damit ermittelten Beatmungs-Betriebspunkt auch eine minimale Beatmungsarbeit geleistet wird. Ebenso soll zur Differenzierung der beiden Hypothesen ausgeschlossen sein, dass an einem aus der Hypothese der minimalen bei der Beatmung geleisteten Arbeit ermittelten Beatmungs-Betriebspunkt gleichzeitig die für die Beatmung aufgewendete Kraft minimal ist. Mit anderen Worten: für gleiche Eingangsgrößen sollen die unterschiedlichen Hypothesen unterschiedliche Beatmungs-Betriebsparameter liefern.

Zur möglichst präzisen Ermittlung eines Beatmungs-Betriebsparameters kann gemäß einer vorteilhaften Weiterbildung der vorliegenden Erfindung vorgesehen sein, dass der erste Datenbasiszusammenhang oder der erste Datenzusammenhang einerseits und der zweite Datenbasiszusammenhang oder der zweite Datenzusammenhang andererseits jeweils einen Zusammenhang einer oder mehrerer Größen aus Patienten-Alveolarvolumen, Patienten-Atemorgane-Totvolumen, Patienten-Atemwege-Strömungswiderstand, Atemgas-Leitungsanordnung-Strömungswiderstand, Patienten-Atemorgane-Nachgiebigkeit, Atemgas-Leitungsanordnung-Nachgiebigkeit und Beatmungsfrequenz oder abgeleiteter bzw. kombinierter Größen einzelner oder mehrerer der vorgenannten Größen enthalten oder bevorzugt angeben. Dabei ist der erste Datenzusammenhang vom zweiten Datenzusammenhang verschieden, auch wenn beide Datenzusammenhänge die gleichen Größen enthalten. "Enthalten" bedeutet dabei, dass neben den oben genannten Größen auch andere Größen in dem Datenzusammenhang berücksichtigt werden können. "Angeben" bedeutet dabei, dass der jeweilige Datenzusammenhang nur aus einer oder mehreren der oben genannten Größen gebildet ist.

Das Patienten-Alveolarvolumen ist dabei dasjenige effektive Volumen, in welchem Gasaustausch zwischen dem in den Patienten eingeleiteten Atemgas und dem Blut des Patienten stattfindet. Das Patienten-Atemorgane-Totvolumen ist dasjenige Volumen der Atemorgane des zu beatmenden Patienten, in dem sich zwar Atemgas befindet und dieses während der Beatmung auch bewegt wird, jedoch das im Totvolumen befindliche Atemgas nicht an einem Gasaustausch mit dem Blut des Patienten teilnimmt. Der Patienten-Atemwege-Strömungswiderstand ist, bevorzugt zusammen mit dem Atemgas-Leitungsanordnung-Strömungswiderstand, die für den jeweiligen Beatmungsfall individuell ermittelte Resistance. Die Patienten-Atemorgane-Nachgiebigkeit ist, bevorzugt zusammen mit der Atemgas-Leitungsanordnung-Nachgiebigkeit, die für den jeweiligen Beatmungsfall einschlägige Compliance.

Somit kann ein Datenbasiszusammenhang aus erstem und zweitem Datenbasiszusammenhang oder ein Datenzusammenhang aus erstem und zweitem Datenzusammenhang ein Zusammenhang der oben genannten Größen bzw. Daten gemäß Mead sein. Ebenso vorteilhaft kann der jeweils andere Datenbasiszusammenhang oder Datenzusammenhang ein Zusammenhang der oben genannten Größen bzw. Daten gemäß Otis sein. Nicht nur haben sich die Formeln gemäß Otis und Mead in der Praxis seit Jahrzehnten bewährt, sie sind darüber hinaus auch weitverbreitet akzeptiert, was für die Akzeptanz einer Beatmungsvorrichtung bei dem damit therapierenden Personal von entscheidender Bedeutung ist.

Grundsätzlich kann daran gedacht sein, ein Patienten-Atemorgane-Teilvolumen, etwa das Patienten-Alveolarvolumen oder/und das Patienten-Atemorgane-Totvolumen, über den vorhandenen Dateneingang in die Steuereinrichtung zu übertragen bzw. einzugeben. Gleiches gilt für den Atemgasbedarf des zu beatmenden Patienten. Da der Atemgasbedarf eines Menschen in dessen Körper verstoffwechselt wird, kann der Atemgasbedarf beispielsweise aus einem Körpergewicht des jeweiligen Patienten hergeleitet werden. Dabei kann sein tatsächliches Körpergewicht verwendet werden oder etwa ein für den jeweiligen Patienten nach an sich bekannten Regeln ermitteltes ideales Körpergewicht (sogenanntes "ideal body weight" = IBW). Je nach Zustand des Patienten kann das zur Ermittlung des Atemgasbedarfs herangezogene Körpergewicht mit einem Koeffizienten multipliziert werden, um daraus etwa ein Minutenvolumen zu errechnen.

Auch das Patienten-Atemorgane-Totvolumen lässt sich in an sich bekannter Weise aus einem Körpergewicht, insbesondere aus dem oben bereits genannten idealen Körpergewicht, ermitteln, etwa wiederum durch Multiplikation mit einen hierfür bestimmten Koeffizienten.

Die vorliegende Erfindung wird nachfolgend anhand der beiliegenden Zeichnungen näher erläutert. Es stellt dar:

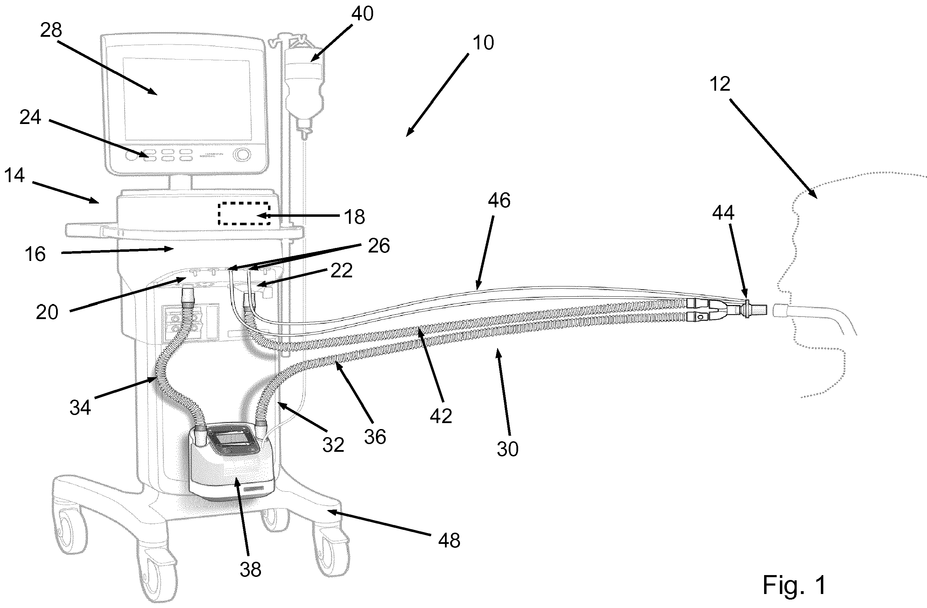

Figur 1    eine schematische Darstellung einer erfindungsgemäßen, zur künstlichen Beatmung eines Patienten hergerichteten Beatmungsvorrichtung,

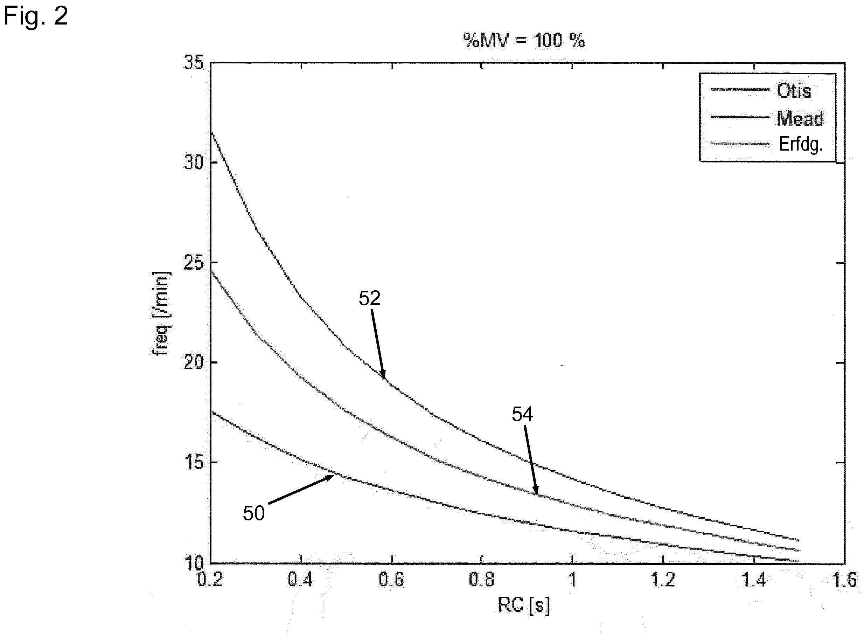

Figur 2    ein von der erfindungsgemäßen Beatmungsvorrichtung beispielhaft verwendeter Zusammenhang von unterschiedlichen Werten der Beatmungs-Zeitkonstante und einer Beatmungsfrequenz als dem Beatmungs-Betriebsparameter und vergleichsweise hierzu entsprechende Zusammenhänge gemäß den Formeln von Otis und Mead für einen ersten Atemgasbedarf,

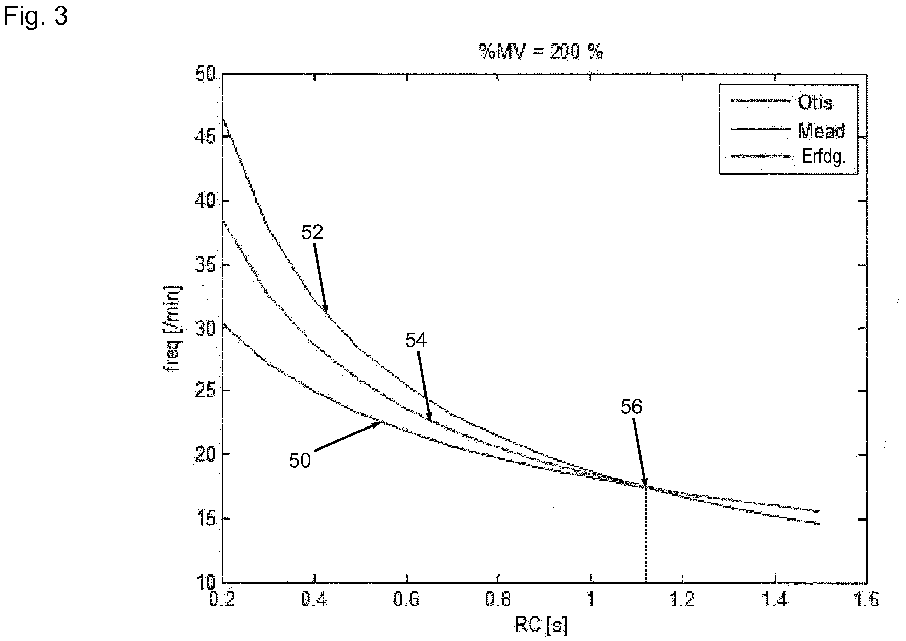

Figur 3   ein von der erfindungsgemäßen Beatmungsvorrichtung beispielhaft verwendeter Zusammenhang von unterschiedlichen Werten der Beatmungs-Zeitkonstante und einer Beatmungsfrequenz als dem Beatmungs-Betriebsparameter und vergleichsweise hierzu entsprechende Zusammenhänge gemäß den Formeln von Otis und Mead für einen zweiten Atemgasbedarf und

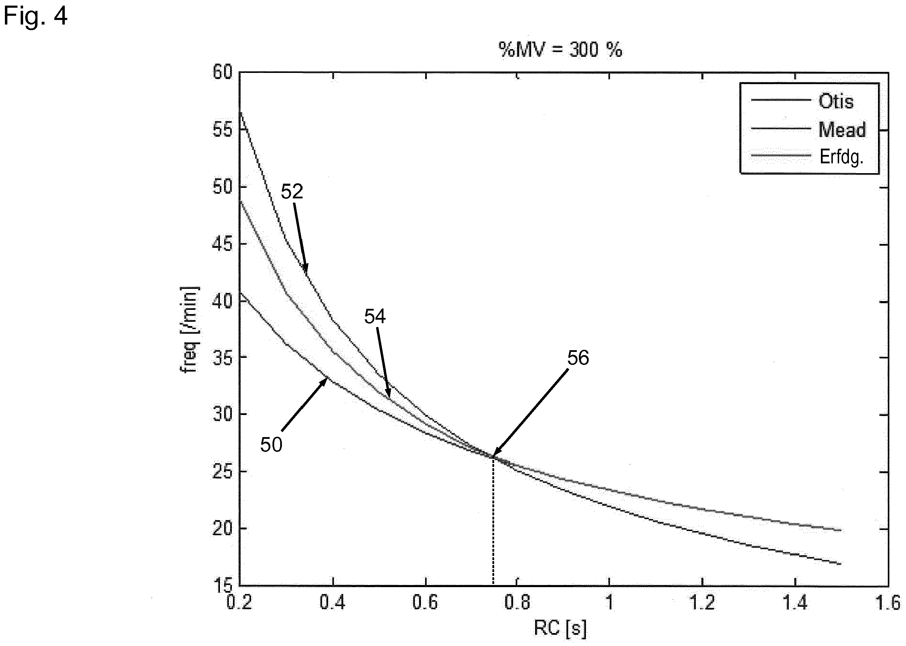

Figur 4   ein von der erfindungsgemäßen Beatmungsvorrichtung beispielhaft verwendeter Zusammenhang von unterschiedlichen Werten der Beatmungs-Zeitkonstante und einer Beatmungsfrequenz als dem Beatmungs-Betriebsparameter und vergleichsweise hierzu entsprechende Zusammenhänge gemäß den Formeln von Otis und Mead für einen dritten Atemgasbedarf.

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12.

Die Beatmungsvorrichtung 10 weist ein Gehäuse 14 auf, in dem - von außen wegen des blickdichten Gehäusematerials nicht erkennbar - eine Druckveränderungsanordnung 16 und eine Steuereinrichtung 18 aufgenommen sein können.

Die Druckveränderungsanordnung 16 ist in an sich bekannter Weise aufgebaut und kann eine Pumpe, einen Verdichter, ein Gebläse, einen Druckbehälter, ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuereinrichtung 18 ist üblicherweise als Computer oder Mikroprozessor realisiert. Sie umfasst eine in Figur 1 nicht dargestellte Speichereinrichtung, um für den Betrieb der Beatmungsvorrichtung 10 notwendige Daten speichern und erforderlichenfalls aufrufen zu können. Die Speichereinrichtung kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist die Speichereinrichtung bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 weist die Beatmungsvorrichtung 10 einen Dateneingang 24 auf, welcher in dem in Figur 1 dargestellten Beispiel durch eine Tastatur repräsentiert ist. Wie weiter unten noch erläutert werden wird, ist die Tastatur nicht der einzige Dateneingang der Steuereinrichtung 18. Tatsächlich kann die Steuereinrichtung 18 Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Druckveränderungsanordnung 16 im Gehäuse 14, über eine Atemgas-Leitungsanordnung 30 verbunden. Der Patient 12 ist hierfür intubiert.

Die Atemgas-Leitungsanordnung 30 weist einen Inspirationsschlauch 32 auf, über welchen frisches Atemgas von der Druckveränderungsanordnung 16 in die Lunge des Patienten 12 geleitet werden kann. Der Inspirationsschlauch 32 kann unterbrochen sein und einen ersten Inspirationsschlauch 34 und einen zweiten Inspirationsschlauch 36 aufweisen, zwischen welchen eine Konditionierungseinrichtung 38 zur gezielten Befeuchtung und gegebenenfalls auch Temperierung des dem Patienten 12 zugeführten frischen Atemgases vorgesehen sein kann. Die Konditionierungseinrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, der Konditionierungseinrichtung 38 zugeführt werden kann. Bei einem Einsatz der vorliegenden Beatmungsvorrichtung 10 als Anästhesie-Beatmungsvorrichtung können so volatile Anästhetika kontrolliert über die Beatmungsvorrichtung 10 an den Patienten 12 abgegeben werden. Die Konditionierungseinrichtung 38 sorgt dafür, dass das frische Atemgas dem Patienten 12 mit einem vorbestimmten Feuchtegehalt, gegebenenfalls unter Zugabe eines Medikamenten-Aerosols und mit einer vorbestimmten Temperatur zugeleitet wird.

Die Atemgas-Leitungsanordnung 30 weist neben dem bereits erwähnten Inspirationsventil 20 und Exspirationsventil 22 weiter einen Exspirationsschlauch 42 auf, über welchen verstoffwechseltes Atemgas aus der Lunge des Patienten 12 in die Atmosphäre abgeblasen wird.

Der Inspirationsschlauch 32 ist mit dem Inspirationsventil 20 gekoppelt, der Exspirationsschlauch 42 mit dem Exspirationsventil 22. Von den beiden Ventilen ist jeweils nur eines gleichzeitig zum Durchlass einer Gasströmung geöffnet. Die Betätigungssteuerung der Ventile 20 und 22 erfolgt ebenfalls durch die Steuereinrichtung 18.

Während eines Beatmungszyklus ist zunächst für die Dauer der Inspirationsphase das Exspirationsventil 22 geschlossen und das Inspirationsventil 20 geöffnet, sodass frisches Atemgas vom Gehäuse 14 zum Patienten 12 geleitet werden kann. Eine Strömung des frischen Atemgases wird durch gezielte Druckerhöhung des Atemgases durch die Druckveränderungsanordnung 16 bewirkt. Aufgrund der Druckerhöhung strömt das frische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Atmosphäre, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck, also einen geringfügig höheren Druck als den Atmosphärendruck, wird die Exspirationsphase mit dem Schließen des Exspirationsventils 22 beendet und es schließt sich ein weiterer Beatmungszyklus an.

Während der Inspirationsphase wird dem Patienten 12 das sogenannte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu kann die Beatmungsvorrichtung 10 mit einem oder mehreren Sensoren datenübertragungsmäßig verbunden sein, welche den Zustand des Patienten oder/und den Betrieb der Beatmungsvorrichtung überwachen. Lediglich beispielhaft für eine Reihe möglicher Sensoren sei in Figur 1 ein Flusssensor 44 genannt, welcher die in der Atemgas-Leitungsanordnung herrschende Atemgas-Strömung erfasst. Der Flusssensor 44 kann mittels einer Sensor-Leitungsanordnung 46 mit den Dateneingängen 26 der Steuereinrichtung 18 gekoppelt sein. Die Sensor-Leitungsanordnung 46 kann, muss jedoch nicht, elektrische Signalübertragungsleitungen umfassen. Sie kann ebenso Schlauchleitungen aufweisen, die den in Strömungsrichtung beiderseits des Flusssensors 44 herrschenden Gasdruck an die Dateneingänge 26 übertragen, wo diese von in Figur 1 nicht dargestellten Drucksensoren quantifiziert werden.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 48 aufgenommen sein kann.

Ist ausgehend von den Patientendaten der Atemgasbedarf des Patienten 12 bekannt - beispielsweise berechnet aus dem idealen Körpergewicht des Patienten 12 unter Berücksichtigung seines Krankheitsbildes -, ist es für eine erfolgreiche Beatmung des Patienten 12 wesentlich, den in der Regel als Minutenvolumen oder Prozent-Minutenvolumen angegebenen Atemgasbedarf in einzelne Atemzüge zu unterteilen.

In Figur 2 sind für ein Prozent-Minutenvolumen von 100 % für den Patienten 12 drei verschiedene Datenzusammenhänge angegeben, die jeweils einen Zusammenhang zwischen der Beatmungs-Zeitkonstante RC und einer Beatmungsfrequenz angeben. Die Beatmungs-Zeitkonstante RC ist gebildet durch das Produkt von Resistance und Compliance. Sie zeigt den Beatmungswiderstand im Zusammenhang mit dem Patienten 12 an. Die Beatmungsfrequenz ist ein Beispiel für einen Beatmungs-Betriebsparameter der Beatmungsvorrichtung 10.

Die Kurve 50 ist dabei auf Grundlage der bekannten Formel von Otis gemäß einer Hypothese minimaler Beatmungsarbeit ermittelt. Sie liefert für das Prozent-Minutenvolumen von 100 % für jeden realistischen Beatmungs-Zeitkonstantenwert die niedrigste Beatmungsfrequenz und damit das größte Beatmungs-Tidalvolumen, da das Minutenvolumen das Produkt aus Beatmungsfrequenz und Beatmungs-Tidalvolumen ist.

Die Kurve 52 in Figur 2 zeigt einen Datenzusammenhang zwischen der Beatmungs-Zeitkonstante RC und der Beatmungsfrequenz, welcher gemäß der bekannten Formel von Mead auf Grundlage einer Hypothese minimaler für die Beatmung aufgewendeter Kraft ermittelt wurde. Dieser Datenzusammenhang gemäß der Formel von Mead liefert für realistische Beatmungs-Zeitkonstantenwerte die größte Beatmungsfrequenz und damit das geringste Beatmungs-Tidalvolumen.

Die erfindungsgemäße Beatmungsvorrichtung 10 kann dabei wie folgt betrieben werden: anhand von sensorisch erfassten Werten, beispielsweise anhand von einer zeitkorrelierten kumulierten Mengenmessung des Atemgases in der Exspirationsphase, kann der aktuell vorliegende Beatmungs-Zeitkonstantenwert ermittelt werden. Der Beatmungs-Zeitkonstantenwert ist per üblicher Definition jene Zeitspanne, die benötigt wird, um 63 % des Beatmungs-Tidalvolumens auszuatmen.

Es existieren jedoch alternativ noch weitere Methoden zur Ermittlung von Zeitkonstanten für einen Fall von künstlicher Beatmung. In diesem Zusammenhang sei auf einen Beitrag von Brunner in "Critical Care Medicine" im Jahr 1995 oder von Lourens in "Intensive Care Medicine" im Jahr 2000 verwiesen. Darin wird die Ermittlung einer exspiratorischen Zeitkonstante als Quotient aus dem Wert von 75% des exspiratorischen Tidalvolumens geteilt durch den exspiratorischen Fluss vorgeschlagen, welcher zum Zeitpunkt des Erreichens einer Exspirationsmenge von 75% des exspiratorischen Tidalvolumens herrscht.

Mit dem so aktuell bestimmten Beatmungs-Zeitkonstantenwert ermittelt die Beatmungsvorrichtung 10, genauer die Steuereinrichtung 18, eine Beatmungsfrequenz nach Mead aus der Kurve 52 als einen ersten Beatmungs-Basisbetriebsparameter und ermittelt eine Beatmungsfrequenz nach Otis aus der Kurve 50 als einen zweiten Beatmungs-Basisbetriebsparameter. Die Kurven 50 und 52 sind daher in dem vorliegenden Beispiel gemäß der obigen Beschreibungseinleitung ein zweiter bzw. ein erster Datenbasiszusammenhang.

In einem nächsten Schritt vergleicht die Beatmungsvorrichtung 10, genauer die Steuereinrichtung 18, den ersten Beatmungs-Basisbetriebsparameter, also die Beatmungsfrequenz nach Mead, mit dem zweiten Beatmungs-Basisbetriebsparameter, also der Beatmungsfrequenz nach Otis. Ist das Ergebnis des Vergleichs, dass der erste Beatmungs-Basisbetriebsparameter nach Mead größer ist als der zweite Beatmungs-Basisbetriebsparameter nach Otis, dann ermittelt die Beatmungsvorrichtung 10 die Beatmungsfrequenz als den Beatmungs-Betriebsparameter als arithmetischen Mittelwert der Beatmungsfrequenzen nach Mead und nach Otis:

$$freq_{Erfindung} = \frac{freq_{Otis} + freq_{Mead}}{2} \qquad\qquad (1)$$

Ist dagegen für den ermittelten Beatmungs-Zeitkonstantenwert die Beatmungsfrequenz nach Otis größer als die Beatmungsfrequenz nach Mead, wird die Beatmungsfrequenz nach Otis als Beatmungs-Basisbetriebsparameter ausgewählt.

Im Beispiel von Figur 1 ist für alle realistisch erreichbaren Beatmungs-Zeitkonstantenwerte die Beatmungsfrequenz nach Otis geringer als jene nach Mead, sodass stets das arithmetische Mittel der Datenzusammenhänge nach Otis und nach Mead als der Datenzusammenhang ausgewählt wird, gemäß dem für ein bekanntes Minutenvolumen und für einen ermittelten Beatmungs-Zeitkonstantenwert eine Beatmungsfrequenz ermittelt wird. Dieser Datenzusammenhang ist in Figur 2 als Kurve 54 bezeichnet.

In Figur 3 sind die im Wesentlichen gleichen Datenbasiszusammenhänge 50 und 52 für ein Prozent-Minutenvolumen von 200 % gezeigt. Es gilt grundsätzlich das oben zu Figur 2 Gesagte auch für Figur 3: Zunächst wird in an sich bekannter Weise der aktuell vorliegende Beatmungs-Zeitkonstantenwert RC ermittelt. Für diesen Beatmungs-Zeitkonstantenwert RC wird je eine Beatmungsfrequenz nach Mead und nach Otis berechnet. Die so erhaltenen Beatmungsfrequenzen nach Mead und nach Otis werden miteinander verglichen. Dann, wenn die Beatmungsfrequenz nach Mead größer ist als jene nach Otis, wird der arithmetische Mittelwert der beiden Beatmungsfrequenzen als der Beatmungs-Betriebsparameter gemäß der oben genannten Formel (1) errechnet. Dann, wenn die Beatmungsfrequenz nach Otis größer ist als jene nach Mead, wird die Beatmungsfrequenz nach Otis als der Beatmungs-Betriebsparameter für den Betrieb der Beatmungsvorrichtung 10 verwendet.

Zu erkennen ist in Figur 3, dass sich die Datenbasiszusammenhänge der Kurven 50 und 52 bei einem Beatmungs-Zeitkonstantenwert von etwa 1,12 s in einem Schnittpunkt 56 schneiden. Somit ist links des Schnittpunkts 56, also bei Beatmungs-Zeitkonstantenwerten kleiner als 1,12 s, die für jeden Beatmungs-Zeitkonstantenwert errechnete Beatmungsfrequenz nach Mead größer als jene nach Otis, sodass für diesen Bereich von Beatmungs-Zeitkonstantenwerten kleiner als 1,12 s stets obige Formel (1) für die Ermittlung der an der Beatmungsvorrichtung 10 als dem Beatmungs-Betriebsparameter einzustellenden Beatmungsfrequenz zur Anwendung kommt.

Für Beatmungs-Zeitkonstantenwerte größer als 1,12 s ist dagegen stets das Gegenteil der Fall: hier ist für jeden Beatmungs-Zeitkonstantenwert die hieraus errechnete Beatmungsfrequenz nach Otis größer als jene nach Mead, sodass für diesen Bereich der Beatmungs-Zeitkonstantenwert stets die Beatmungsfrequenz nach Otis als der Beatmungs-Betriebsparameter an der Beatmungsvorrichtung 10 eingestellt bzw. verwendet wird. Der Schnittpunkt 56 ist ein Schwellen-Widerstandsdatenwert, wie er oben in der Beschreibungseinleitung genannt ist.

Figur 4 zeigt im Wesentlichen die gleichen prinzipiellen Datenzusammenhänge bzw. Datenbasiszusammenhänge wie die Figuren 2 und 3, jedoch für ein Prozent-Minutenvolumen von 300 % also für einen stark erhöhten Atemgasbedarf. Es gilt für Figur 4 mutatis mutandis das zu Figur 3 Gesagte mit der Maßgabe, dass der Schnittpunkt bzw. Schwellen-Widerstandsdatenwert 56 für den in Figur 4 einschlägigen Atemgasbedarf bei einem Beatmungs-Zeitkonstantenwert von 0,75 s liegt.

Mit der Beatmungsvorrichtung 10 der vorliegenden Erfindung kann die an Patienten 12 durchgeführte künstliche Beatmung besser als bisher auf den konkreten Patienten und seinen Zustand abgestimmt werden. Da sowohl das Berechnungsverfahren nach Otis wie das Berechnungsverfahren nach Mead weithin anerkannt und akzeptiert sind, kann auch einem Wert, welcher zwischen den Ergebnissen der beiden bekannten Berechnungsverfahren gelegen ist, die Anerkennung der Anwendbarkeit nicht versagt werden. Denn wenn jeder der aus Otis und Mead für einen Beatmungs-Zeitkonstantenwert und für einen konkreten Atemgasbedarf erhaltene Beatmungs-Betriebsparameter therapeutisch tauglich ist, muss auch ein zwischen diesen bekannten Beatmungs-Betriebsparametern gelegener neuer Beatmungs-Betriebsparameter therapeutisch tauglich sein.

**Patentansprüche**

1. Beatmungsvorrichtung (10) zur wenigstens unterstützend-teilweisen künstlichen Beatmung von Patienten (12), insbesondere von humanen Patienten, mit einer Atemgas-Leitungsanordnung (30), mit einer Druckveränderungsanordnung (16) zur Veränderung des Drucks von Atemgas in der Atemgas-Leitungsanordnung (30) während des Beatmungsbetriebs der Beatmungsvorrichtung (10) und mit einer Steuereinrichtung (18), welche die Druckveränderungsanordnung (16) derart steuert, dass dem Patienten ein vorgegebenes Atemgas-Minutenvolumen zugeführt wird, wobei die Steuereinrichtung (18) einen Dateneingang (24, 26) zur Übertragung von Betriebs- oder/und Patientendaten an die Steuereinrichtung (18) aufweist und wobei die Steuereinrichtung (18) dazu ausgebildet ist, ein Beatmungs-Tidalvolumen oder eine Beatmungsfrequenz als einen Beatmungs-Betriebsparameter für den Betrieb der Druckveränderungsanordnung (16) selektiv mittels eines vorbestimmten ersten Datenzusammenhangs (54) oder mittels eines vom ersten verschiedenen vorbestimmten zweiten Datenzusammenhangs (50) zu ermitteln, **dadurch gekennzeichnet, dass** die Steuereinrichtung (18) dazu ausgebildet ist, abhängig von einem einen Beatmungswiderstand im Zusammenhang mit einem zu beatmenden Patienten (12) anzeigenden Widerstandsdatenwert einen Datenzusammenhang (50, 54) aus dem vorbestimmten ersten (54) und dem vorbestimmten zweiten Datenzusammenhang (50) auszuwählen und so den Beatmungs-Betriebsparameter für das vorgegebene Atemgas-Minutenvolumen gemäß dem ausgewählten Datenzusammenhang (50, 54) aus erstem Datenzusammenhang (54) und zweitem Datenzusammenhang (50) zu ermitteln.

2. Beatmungsvorrichtung (10) nach Anspruch 1, **dadurch gekennzeichnet, dass** die Beatmungsvorrichtung (10) dazu ausgebildet ist, den Widerstandsdatenwert zu bestimmen, bevorzugt wiederholt zu bestimmen, besonders bevorzugt nach jedem oder für jeden Beatmungszyklus zu bestimmen, wobei die Beatmungsvorrichtung (10) hierzu bevorzugt mit einem oder mehreren Sensoren zur Erfassung von Betriebsgrößen der Druckveränderungsanordnung (16) oder/und der Atemgas-Leitungsanordnung (30) datenübertragungsmäßig verbunden sein kann.

3. Beatmungsvorrichtung (10) nach Anspruch 1 oder 2, **dadurch gekennzeichnet, dass** die Beatmungsvorrichtung (10) dazu ausgebildet ist, für einen Widerstandsdatenwert einen ersten Beatmungs-Basisbetriebsparameter nach einem vorbestimmten ersten Datenbasiszusammenhang (52) zu bestimmen und einen zweiten Beatmungs-Basisbetriebsparameter nach einem vom ersten verschiedenen vorbestimmten zweiten Datenbasiszusammenhang (50) zu bestimmen, wobei die Beatmungsvorrichtung (10) weiter dazu ausgebildet ist, den ersten und den zweiten Beatmungs-Basisbetriebsparameter miteinander zu vergleichen und abhängig von einem Ergebnis des Vergleichs den Beatmungs-Betriebsparameter mittels des vorbestimmten ersten Datenzusammenhangs (54) oder mittels des vorbestimmten zweiten Datenzusammenhangs (50) zu ermitteln.

4. Beatmungsvorrichtung (10) nach Anspruch 3, **dadurch gekennzeichnet, dass** der erste Datenzusammenhang (54) eine Funktion des ersten (52) oder/und des zweiten Datenbasiszusammenhangs (50) ist, vorzugsweise eine einen Mittelwert, besonders bevorzugt einen arithmetischen Mittelwert, aus dem ersten (52) und dem zweiten Datenbasiszusammenhang (50) bildende Funktion ist und dass der zweite Datenzusammenhang (50) eine Funktion des ersten (52) oder/und des zweiten Datenbasiszusammenhangs (50) ist, vorzugsweise der zweite Datenbasiszusammenhang (50) ist.

5. Beatmungsvorrichtung (10) nach Anspruch 3 oder 4, **dadurch gekennzeichnet, dass** die Beatmungsvorrichtung (10) dazu ausgebildet ist, dann, wenn der erste Beatmungs-Basisbetriebsparameter größer als der zweite Beatmungs-Basisbetriebsparameter ist, einen Mittelwert, vorzugsweise arithmetischen Mittelwert, des ersten und des zweiten Beatmungs-Basisbetriebsparameters als den Beatmungs-Betriebsparameter zu verwenden, und dann, wenn der erste Beatmungs-Basisbetriebsparameter kleiner als der zweite Beatmungs-Basisbetriebsparameter ist oder gleich dem Beatmungs-Basisbetriebsparameter ist, den zweiten Beatmungs-Basisbetriebsparameter ist als den Beatmungs-Betriebsparameter zu verwenden.

6. Beatmungsvorrichtung (10) nach einem der vorhergehenden Ansprüche,
**dadurch gekennzeichnet, dass** für wenigstens einen Teil von an der Beatmungsvorrichtung (10) auswählbaren oder/und einstellbaren Atemgasbedarfswerten jeweils ein Schwellen-Widerstandsdatenwert (56) existiert, wobei die Steuereinrichtung (18) dazu ausgebildet ist, dann, wenn der Widerstandsdatenwert des zu beatmenden Patienten (12) unterhalb des Schwellen-Widerstandsdatenwerts (56) gelegen ist, den Beatmungs-Betriebsparameter gemäß dem ersten Datenzusammenhang (54), bevorzugt in Form einer Funktion eines ersten (52) oder/und eines zweiten Datenbasiszusammenhangs (50), zu ermitteln, und dann, wenn der Widerstandsdatenwert des konkreten Patienten (12) oberhalb des Schwellen-Widerstandsdatenwerts (56) gelegen ist, den Beatmungs-Betriebsparameter gemäß dem zweiten Datenzusammenhang (50), insbesondere bevorzugt in Form einer Funktion eines ersten (52) oder/und eines zweiten Datenbasiszusammenhangs (50), zu ermitteln.

7. Beatmungsvorrichtung (10) nach Anspruch 6,
**dadurch gekennzeichnet, dass** der erste (54) und der zweite Datenzusammenhang (50) bei ansonsten gleichen Betriebs- und Patientendaten für den Schwellen-Widerstandsdatenwert (56) die Ermittlung im Wesentlichen desselben Werts für den Beatmungs-Betriebsparameter bewirken.

8. Beatmungsvorrichtung (10) nach einem der vorhergehenden Ansprüche,
**dadurch gekennzeichnet, dass** der Atemgasbedarf in Prozent-Minutenvolumen angegeben ist.

9. Beatmungsvorrichtung (10) nach einem der vorhergehenden Ansprüche,
**dadurch gekennzeichnet, dass** der Widerstandsdatenwert einen Strömungswiderstandswert (Resistance) der Atemwege des zu beatmenden Patienten (12) oder/und der Atemgas-Leitungsanordnung (30) oder/und einen Nachgiebigkeitswert (Compliance) der Atmungsorgane des zu beatmenden Patienten (12) oder/und der Atemgas-Leitungsanordnung (30) berücksichtigt, wobei der Widerstandsdatenwert bevorzugt eine durch das Produkt von Strömungswiderstandswert (Resistance) und Nachgiebigkeitswert (Compliance) im Zusammenhang mit dem zu beatmenden Patienten (12) gebildete Beatmungs-Zeitkonstante berücksichtigt, höchst bevorzugt die Beatmungs-Zeitkonstante ist.

10. Beatmungsvorrichtung (10) nach einem der vorhergehenden Ansprüche,
**dadurch gekennzeichnet, dass** der erste Datenbasiszusammenhang (52) oder der erste Datenzusammenhang (54) auf einer Hypothese minimalen Kraftaufwands für die Beatmung ohne gleichzeitig minimaler bei der Beatmung geleisteter Arbeit beruht und dass der zweite Datenbasiszusammenhang (50) oder der zweite Datenzusammenhang (50) auf einer Hypothese minimaler bei der Beatmung geleisteter Arbeit ohne gleichzeitig minimaler für die Beatmung aufgewendeter Kraft beruht.

11. Beatmungsvorrichtung (10) nach einem der vorhergehenden Ansprüche,
**dadurch gekennzeichnet, dass** der erste Datenbasiszusammenhang (52) oder der erste Datenzusammenhang (54) einerseits und der zweite Datenbasiszusammenhang (50) oder der zweite Datenzusammenhang (50) andererseits jeweils einen Zusammenhang von einzelnen oder mehreren Größen aus Patienten-Alveolarvolumen, Patienten-Atemorgane-Totvolumen, Patienten-Atemwege-Strömungswiderstand, Patienten-Atemorgane-Nachgiebigkeit, Atemgas-Leitungsanordnung-Strömungswiderstand, Atemgas-Leitungsanordnung-Nachgiebigkeit und Beatmungsfrequenz oder abgeleiteter bzw. kombinierter Größen einzelner oder mehrerer der vorgenannten Größen enthalten oder bevorzugt angeben, wobei besonders bevorzugt der erste Datenbasiszusammenhang (52) oder der erste Datenzusammenhang (54) ein Zusammenhang der genannten Daten gemäß Mead ist und wobei besonders bevorzugt der zweite Datenbasiszusammenhang (50) oder der zweite Datenzusammenhang (50) ein Zusammenhang der genannten Daten gemäß Otis ist.

12. Beatmungsvorrichtung (10) nach einem der vorhergehenden Ansprüche,
**dadurch gekennzeichnet, dass** die Steuereinrichtung (18) dazu ausgebildet ist, abhängig von Patientendaten eines zu beatmenden Patienten (12) ein Patienten-Atemorgane-Teilvolumen oder/und einen Atemgasbedarf zu ermitteln.

13. Beatmungsvorrichtung (10) nach Anspruch 12,
**dadurch gekennzeichnet, dass** die Steuereinrichtung (18) dazu ausgebildet ist, ein Patienten-Atemorgan-Totvolumen in Abhängigkeit von einem idealen Körpergewicht des zu beatmenden Patienten (12) zu ermitteln oder/und dazu ausgebildet ist, ein Minutenvolumen in Abhängigkeit von einem idealen Körpergewicht des zu beatmenden Patienten (12) zu ermitteln.

**Claims**

1. Respiratory device (10) for the at least supportive-partial artificial respiration of patients (12), in particular human patients, comprising a respiratory gas conduit assembly (30), a pressure changing assembly (16) for changing the pressure of respiratory gas in the respiratory gas conduit assembly (30) during the respiratory operation of the respiratory device (10), and a control device (18), which controls the respiratory operation of the respiratory device (10), in particular of the pressure changing assembly (16) such that a predetermined respiratory gas minute volume is supplied to the patient, wherein the control device (18) has a data input (24, 26) for transmitting operational and/or patient data to the control device (18), and wherein the control device (18) is configured to selectively determine respiration tidal volume or a respiration frequency as a respiratory operating parameter for the operation of the pressure changing assembly (16) using a predetermined first data relationship (54) or using a predetermined second data relationship (50) that is different from the first data relationship,
**characterized in that** the control device (18) is configured to select a data relationship (50, 54) from the predetermined first (54) and the predetermined second data relationship (50) depending on a resistance data value denoting a respiration resistance in connection with a patient (12) to be ventilated, and thus to determine the respiratory operating parameter for the predetermined respiratory gas minute volume according to the selected data relationship (50, 54) from the first data relationship (54) and the second data relationship (50).

2. Respiratory device (10) according to claim 1, **characterized in that** the respiratory device (10) is configured to determine the resistance data value, preferably repeatedly, particularly preferably after each or for each respiratory cycle, wherein the respiratory device (10) can be connected preferably to one or to a plurality of sensors to determine operating parameters of the pressure changing arrangement (16) and/or of the respiratory gas conduit assembly (30) for the purpose of data transmission.

3. Respiratory device (10) according to claim 1 or 2, **characterized in that** the respiratory device (10) is configured to determine a first respiratory base operating parameter for a resistance data value according to a predetermined, first data base relationship (52) and to determine a second respiratory base operating parameter according to a predetermined, second data base relationship (50) different from the first, wherein the respiratory device (10) is further configured to compare the first and the second respiratory base operating parameters with each other, and depending on the result of the comparison, to determine the respiratory operating parameter using the predetermined, first data relationship (54) or using the predetermined, second data relationship (50).

4. Respiratory device (10) according to claim 3, **characterized in that** the first data relationship (54) is a function of the first (52) and/or of the second data base relationship (50), preferably a function forming an average value, quite preferably an arithmetic average value, of the first (52) and the second data base relationship (50), and that the second data relationship (50) is a function of the first (52) and/or of the second data base relationship (50), preferably of the second data base relationship (50).

5. Respiratory device (10) according to claim 3 or 4, **characterized in that** the respiratory device (10) is configured such that when the first respiratory base operating parameter is greater than the second respiratory base operating parameter, an average value, preferably an arithmetic average value, of the first and of the second respiratory base operating parameter is used as the respiratory operating parameter, and when the first respiratory base operating parameter is less than the second respiratory base operating parameter or is equal to the respiratory base operating parameter, the second respiratory base operating parameter is used as the respiratory operating parameter.

6. Respiratory device (10) according to one of the preceding claims, **characterized in that** for at least a portion of the respiratory gas requirements selectable and/or adjustable on the respiratory device (10), there exists one threshold resistance data value (56) each, wherein the control device (18) is configured such that when the resistance data value of the patient (12) to be ventilated is below the threshold resistance data value (56), the respiratory device determines the respiration operating parameter according to the first data relationship (54), preferably in the form of a function of a first (52) and/or of a second data base relationship (50), and when the resistance data value of the concrete patient (12) is above the threshold resistance data value (56), the respiratory device determines the respiration operating parameter according to the second data relationship (50), especially preferably in the form of a function of a first (52) and/or of a second data base relationship (50).

7. Respiratory device (10) according to claim 6, **characterized in that** when the operating and patient data for the threshold resistance data value (56) are otherwise equal, then the first (54) and the second data relationship (50) cause the determination of essentially the same value for the respiration operating parameter.

**8.** Respiratory device (10) according to one of the preceding claims, **characterized in that** the respiration gas requirement is stated in percent minute volume.

**9.** Respiratory device (10) according to one of the preceding claims, **characterized in that** the resistance data value takes into account a flow resistance value (resistance) of the respiratory path of the patient (12) to be ventilated and/or of the respiration gas conduit assembly (30), and/or a flexibility value (compliance) of the respiration organs of the patient (12) to be ventilated and/or of the respiration gas conduit assembly (30), wherein the resistance data value takes into account preferably a respiration time-constant formed by the product of flow resistance value (resistance) and flexibility value (compliance) in connection with the patient (12) to be ventilated, and most preferably is the respiration-time constant.

**10.** Respiratory device (10) according to one of the preceding claims, **characterized in that** the first data base relationship (52) or the first data relationship (54) is based on a hypothesis of a minimum of force applied for the respiration, without simultaneously applying a minimum of work for the respiration, and that the second data base relationship (50) or the second data relationship (50) is based on a hypothesis of a minimum of work performed for the respiration, without simultaneously applying a minimum of force for the respiration.

**11.** Respiratory device (10) according to one of the preceding claims, **characterized in that** the first data base relationship (52) or the first data relationship (54) on the one hand, and the second data base relationship (50) or the second data relationship (50) on the other hand, each contains or preferably defines a relationship of individual or a plurality of quantities of patient alveolar volume, patient respiratory organ dead-volume, patient respiratory path flow resistance, patient respiratory organ compliance, respiratory gas conduit arrangement flow resistance, respiratory gas conduit arrangement compliance and respiration frequency or derived or combined quantities of individual or a plurality of the aforestated quantities, wherein particularly preferably the first data base relationship (52) or the first data relationship (54) is a relationship of the stated data pursuant to Mead, and wherein particularly preferably the second data base relationship (50) or the second data relationship (50) is a relationship of the stated data pursuant to Otis.

**12.** Respiratory device (10) according to one of the preceding claims, **characterized in that** the control device (18) is configured to determine a patient respiratory organ part volume and/or a respiratory gas requirement, depending on patient data of a patient (12) to be ventilated.

**13.** Respiratory device (10) according to claim 12, **characterized in that** the control device (18) is configured to determine a patient respiratory organ dead volume as a function of an ideal body weight of the patient (12) to be ventilated and/or is configured to determine a volume per minute as a function of an ideal body weight of the patient (12) to be ventilated.

**Revendications**

**1.** Appareil respiratoire (10) pour la ventilation au moins partiellement assistée de patients (12), en particulier de patients humains, avec un arrangement de conduite de gaz respiratoire (30), avec un arrangement de modification de pression (16) pour modifier la pression de gaz respiratoire dans l'arrangement de conduite de gaz respiratoire (30) pendant l'opération de ventilation artificielle de l'appareil respiratoire (10) et avec un dispositif de commande (18) commandant l'arrangement de modification de pression (16) de sorte qu'un volume minute de gaz respiratoire est fourni au patient, où le dispositif de commande (18) comprend une entrée de données (24, 26) pour transmettre des données opératoires ou/et des patients au dispositif de commande (18) et où le dispositif de commande (18) est adapté pour déterminer sélectivement un volume courant de ventilation ou une fréquence de ventilation en tant que paramètre opératoire de ventilation pour l'opération de l'arrangement de modification de pression (16) au moyen d'une première relation de données prédéterminée (54) ou au moyen d'une deuxième relation de données prédéterminée (50) différente de la première relation,
**caractérisé en ce que** le dispositif de commande (18) est adapté pour sélecter une relation de données (50, 54) de la première relation de données prédéterminée (54) et de la deuxième relation de données prédéterminée (50) en fonction d'une valeur de données de résistance indiquant une résistance respiratoire dans le contexte d'un patient à ventiler (12) et pour ainsi déterminer le paramètre opératoire de ventilation pour le volume minute de gaz respiratoire prédéterminé selon la relation de données (50, 54) sélectée de la première relation de données prédéterminée (54) et la deuxième relation de données prédéterminée (50).

**2.** Appareil respiratoire (10) selon la revendication 1,
**caractérise en ce que** l'appareil respiratoire (10) est adapté pour déterminer la valeur de données de résistance, de préférence de manière répétitive, de manière particulièrement préférée après chaque cycle respiratoire ou pour chaque cycle respiratoire, où l'appareil respiratoire (10) peut à cette fin de préférence être connecté à un ou plusieurs capteurs pour détecter des paramètres de l'arrangement de modification de pression (16) ou/et de l'arrangement de conduite de gaz respiratoire (30) pour une transmission de données.

**3.** Appareil respiratoire (10) selon la revendication 1 ou 2,
**caractérise en ce que** l'appareil respiratoire (10) est adapté pour déterminer un premier paramètre d'opération respiratoire de base pour une valeur de données de résistance selon une première relation de base de données prédéterminée (52) et pour déterminer un deuxième paramètre d'opération respiratoire de base selon une deuxième relation de base de données prédéterminée (50) différente de la première relation, l'appareil respiratoire (10) étant en outre adapté pour comparer le premier au deuxième paramètre d'opération respiratoire de base et pour déterminer en fonction d'un résultat de la comparaison le paramètre d'opération respiratoire au moyen de la première relation de données prédéterminée (54) ou au moyen de la deuxième relation de données prédéterminée (50).

**4.** Appareil respiratoire (10) selon la revendication 3,
**caractérise en ce que** la première relation de données (54) est une fonction de la première relation de base de données (52) ou/et de la deuxième relation de base de données (50), de préférence une fonction formant une moyenne, de manière particulièrement préférée une moyenne arithmétique, de la première (52) et de la deuxième relation de base de données (50) et **en ce que** la deuxième relation de base de données (50) est une fonction de la première (52) ou/et de la deuxième relation de base de données (50), et est de préférence la deuxième relation de base de données (50).

**5.** Appareil respiratoire (10) selon la revendication 3 ou 4,
**caractérise en ce que** l'appareil respiratoire (10) est adapté pour utiliser une moyenne, de préférence une moyenne arithmétique, de la première et de la deuxième paramètre d'opération respiratoire de base comme paramètre d'opération respiratoire quand le première paramètre d'opération respiratoire de base est plus grand que le deuxième paramètre d'opération respiratoire de base, et pour utiliser le deuxième paramètre d'opération respiratoire de base comme paramètre d'opération respiratoire quand le premier paramètre d'opération respiratoire de base est plus petit que le deuxième paramètre d'opération respiratoire de base ou est égal au paramètre d'opération respiratoire de base.

**6.** Appareil respiratoire (10) selon une des revendications précédentes,
**caractérise en ce que** pour au moins une partie des valeurs des besoins de gaz respiratoire sélectables ou/et réglables à l'appareil respiratoire (10) il y a une valeur limite de résistance (56) respective, le dispositif de commande (18) étant adapté pour déterminer le paramètre d'opération respiratoire selon la première relation de données (54), de préférence sous forme d'une fonction d'une première (52) ou/et d'une deuxième relation de base de données (50), quand la valeur de résistance du patient à ventiler (12) est inférieur à la valeur limite de résistance (56), et pour déterminer le paramètre d'opération respiratoire selon la deuxième relation de données (50), de préférence sous forme d'une fonction d'une première (52) ou/et d'une deuxième relation de base de données (50), quand la valeur de résistance du patient à ventiler concret (12) est supérieur à la valeur limite de résistance (56).

**7.** Appareil respiratoire (10) selon la revendication 6,
**caractérise en ce que** la première (54) et la deuxième relation de données (50) mènent en cas des autrement mêmes données opératoires et données des patients pour la valeur limite de résistance (56) à la détermination d'essentiellement la même valeur pour le paramètre d'opération respiratoire.

**8.** Appareil respiratoire (10) selon une des revendications précédentes,
**caractérise en ce que** le besoin de gaz respiratoire est précisé en pourcentage de volume par minute.

**9.** Appareil respiratoire (10) selon une des revendications précédentes,
**caractérise en ce que** la valeur de données de résistance prend en compte une valeur de résistance (Résistance) au flux des voies respiratoires du patient à ventiler (12) ou/et de l'arrangement de conduite de gaz respiratoire (30) ou/et une valeur de conformité (Compliance) des organes respiratoires du patient à ventiler (12) ou/et de l'arrangement de conduite de gaz respiratoire (30), la valeur de données de résistance prenant de préférence en compte une constante de temps de ventilation formée par le produit de la valeur de résistance (Résistance) au flux et de la valeur de conformité (Compliance) dans le contexte du patient à ventiler (12).

**10.** Appareil respiratoire (10) selon une des revendications précédentes, **caractérise en ce que** la première relation de base de données (52) ou la première relation de données (54) sont basées sur une hypothèse d'effort minimal pour la ventilation sans travail minimal simultané effectué pendant la ventilation et **en ce que** la deuxième relation de base de données (50) ou la deuxième relation de données (50) est basée sur une hypothèse de travail minimal effectué pour la ventilation sans force minimal simultané exercée pendant la ventilation.

**11.** Appareil respiratoire (10) selon une des revendications précédentes, **caractérise en ce que** la première relation de base de données (52) ou la première relation de données (54) d'un côté et la deuxième relation de base de données (50) ou la deuxième relation de données (50) d'autre côté contiennent ou comprennent de préférence un rapport d'un ou de plusieurs paramètres parmi volume alvéolaire du patient, volume mort d'organes respiratoires du patient, résistance au flux d'organes respiratoires du patient, flexibilité d'organes respiratoires du patient, résistance au flux de l'arrangement de conduite de gaz respiratoire, flexibilité de l'arrangement de conduite de gaz respiratoire et fréquence respiratoire ou des paramètres dérivés ou combinés d'un ou de plusieurs paramètres précités, où de manière particulièrement préférée la première relation de base de données (52) ou la première relation de données (54) est une relation des données précitées selon Mead et où de manière particulièrement préférée la deuxième relation de base de données (50) ou la deuxième relation de données (50) est une relation des données précitées selon Otis.

**12.** Appareil respiratoire (10) selon une des revendications précédentes, **caractérise en ce que** le dispositif de commande (18) est adapté pour déterminer un volume partiel d'organes respiratoire du patient ou/et un besoin de gaz respiratoire en fonction des données de patient d'un patient à ventiler (12).

**13.** Appareil respiratoire (10) selon la revendication 12, **caractérise en ce que** le dispositif de commande (18) est adapté pour déterminer un volume mort d'organes respiratoires du patient en fonction d'un poids idéal du patient à ventiler (12) ou/et est adapté pour déterminer un volume minute en fonction d'un poids idéal du patient à ventiler (12).

Fig. 1

Fig. 2

Fig. 3

%MV = 200 %

Fig. 4

%MV = 300 %

EP 3 261 698 B1

**IN DER BESCHREIBUNG AUFGEFÜHRTE DOKUMENTE**

**In der Beschreibung aufgeführte Patentdokumente**

- US 2013074844 A1
- US 2012298108 A1
- US 2009007915 A1
- US 2012272961 A1
- WO 2007085108 A
- WO 2013045563 A1

**In der Beschreibung aufgeführte Nicht-Patentliteratur**

- **BEITRAG VON BRUNNER.** *Critical Care Medicine,* 1995
- **VON LOURENS.** *Intensive Care Medicine,* 2000